# EUROPEAN PATENT SPECIFICATION

(11) **EP 2 888 996 B1**
(45) Date of publication and mention of the grant of the patent: **31.05.2017**
(21) Application number: 14200053.8
(22) Date of filing: 23.12.2014
(51) Int. Cl.: A61B 5/01, A61B 5/11, A61B 5/1455, A61B 5/0205, A61B 5/024, A61B 5/00

(54) **Biometric information detecting apparatus**
Biometrische Datenerkennungsvorrichtung
Appareil de détection d'informations biométriques

(30) Priority: 25.12.2013 JP 2013266631
(43) Date of publication of application: 01.07.2015
(73) Proprietor: Seiko Epson Corporation, Shinjuku-ku Tokyo 163-0811 (JP)
(72) Inventor: Shimizu, Yoshiaki, Nagano, 392-8502 (JP)
(74) Representative: Hoffmann Eitle

(56) References cited:
- US-A1- 2008 167 539
- US-A1- 2013 187 789

## Description

### BACKGROUND

### 1. Technical Field

The present invention relates to a biometric information detecting apparatus.

### 2. Related Art

In the related art, a biometric information detecting apparatus that detects biometric information from person's pulse or the like is known. JP-A-2008-167893, JP-A-2008-168054, and JP-A-2006-312010 disclose a related art pulsimeter that is an example of the biometric information detecting apparatus. The pulsimeter is mounted on the wrist, arm, finger or the like, for example, to detect pulsation due to the human heartbeat, and to measure the pulse rate.

However, a biometric information detecting apparatus (pulsimeter or the like) capable of continuously measuring biometric information with a small size and a light weight without having an influence on daily life of the wearer even during long wearing of the biometric information detecting apparatus has not yet been proposed.

Further, a pleasant appearance is an important factor in daily use, but up to now, the biometric information detecting apparatus has had greater importance attached to satisfaction of performance specifications, and has had less importance attached to the pleasant appearance thereof.

For example, JP-A-2008-167893 discloses a biometric information detecting apparatus which is worn on a finger. However, in this biometric information detecting apparatus, the finger is difficult to use when wearing the apparatus, which may cause difficulties in daily life.

JP-A-2008-168054 discloses a biometric information detecting apparatus which is a bracelet. However, since the biometric information detecting apparatus is not wound around the wrist when wearing the apparatus, if the biometric information detecting apparatus comes into contact with an obstacle during a wild action such as swinging of an arm, the biometric information detecting apparatus may deviate from its position.

JP-A-2006-312010 discloses a biometric information detecting apparatus which is a watch. However, in this biometric information detecting apparatus, since a display unit such as an LCD is provided, approximately the same size required for a wrist watch is necessary, which may cause a user to feel uncomfortable during long wearing of the apparatus fastened to the wrist for detection of biometric information.

US 2008/167539 A1 is concerned with an apparatus for measuring a state parameter of an individual using signals based on one or more sensors.

### SUMMARY

An advantage of some aspects of the invention is to provide a biometric information detecting apparatus capable of detecting biometric information continuously while reducing the burden during long wearing of the apparatus.

The invention is defined in claim 1 and can be implemented as the following forms or application examples.

An aspect of the invention relates to a biometric information detecting apparatus is further defined in the claims.

According to this aspect of the invention, the sensor unit is provided in the case unit, and the biometric information is detected based on the detection signal from the sensor unit. Further, the band unit includes the inner surface having the first curved surface shape on the side thereof that faces the test object when wearing the apparatus, and the case unit attached to the band unit includes the outer surface having unit that faces the inner surface in the curved surface shape, the thickness of the case unit can be made to be thin, and a small and light biometric information detecting apparatus can be realized. Thus, a biometric information detecting apparatus capable of detecting biometric information continuously while reducing the burden during long wearing of the apparatus can be provided.

In the aspect of the invention, the case unit may include a top case and a bottom case, and at least the top case and the band unit may be insert-molded.

With such a configuration using insert-molding, the band unit and the top case may be formed of optimal materials, respectively. In addition, during insert-molding, a manufacturing process of attaching the top case to the band unit is not necessary, to thereby simplify the manufacturing process, and the biometric information detecting apparatus can be provided at low cost.

In the aspect of the invention, the band unit may be formed of a first material, and the top case may be formed of a second material harder than the first material.

With such a configuration, as the band unit is formed of the soft first material compared with the top case, a good fit when wearing the apparatus can be provided. Further, as the top case is formed of the hard second material, breakage, failure or the like of the built-in components of the case unit can be suppressed.

In the aspect of the invention, the case unit may include a top case and a bottom case, and a hole portion that accommodates the top case may be provided in the band unit.

With such a configuration, for example, in a state where the top case is accommodated in the hole portion so that the top case is covered by the band unit, the band unit and the top case can be integrally formed.

In the aspect of the invention, a flexible substrate on which a light emitting portion is disposed may be provided on a rear surface side of the outer surface of the case unit.

With such a configuration, notification of a variety of information using the light emitting portion can be performed. Further, since the light emitting portion is mounted on the flexible substrate, the thickness of the case unit can be made to be thin, for example.

In the aspect of the invention, the case unit may include a top case and a bottom case, the top case may include a light emitting window portion for the light emitting portion, and the top case and the light emitting window portion may be insert-molded.

In this way, if the top case and the light emitting window portion are insert-molded, the top case and the light emitting window portion can be formed using a simple manufacturing process with a small amount of man-hours, and the biometric information detecting apparatus can be provided at low cost.

In the aspect of the invention, a flexible substrate on which a communication antenna is disposed may be provided on a rear surface of the outer surface of the case unit.

In this way, if the antenna is formed on the flexible substrate, the antenna can be disposed at a position close to the outer surface of the case unit, and reception sensitivity of the antenna can be enhanced, for example.

In the aspect of the invention, a circuit board on which the processing unit is mounted may be provided in the case unit, the case unit may include a top case and a bottom case, and a secondary battery may be disposed between the circuit board and the top case.

With such a configuration, the empty space of the circuit board on the side of the top case can be effectively used for disposition of the secondary battery, and thus, the case unit can be made to be thin and small, for example.

In the aspect of the invention, a circuit board on which the processing unit is mounted and a vibration generator may be provided in the case unit, and the vibration generator may be provided, among a first side and a second side of the vibration generator that are opposite to each other, on the first side.

With such a configuration, the empty space on the first side of the case unit can be effectively used for disposition of the vibration generator, and thus, the component arrangement in which the space in the case unit is effectively used can be achieved and the case unit can be made to be thin and small.

In the aspect of the invention, a body motion sensor unit may be mounted on the circuit board, and a distance between the vibration generator and the body motion sensor unit may be greater than a distance between the vibration generator and the processing unit.

With such a configuration, the distance between the vibration generator and the body motion sensor unit can be increased, and thus, the negative influence of vibration generated in the vibration generator on the body motion sensor unit can be reduced.

In the aspect of the invention, a vibration generator is provided in the case unit, the case unit includes a top case and a bottom case, and a shock absorber is provided between the vibration generator and the bottom case.

In this way, if the shock absorber is provided, a variety of information can be notified from the vibration generator using appropriate vibration from which an uncomfortable vibration or the like is removed.

In the aspect of the invention, a circuit board on which the processing unit is mounted and a sensor substrate on which the sensor unit is mounted is provided in the case unit, and a shock absorber is provided between the circuit board and the sensor substrate.

In this way, if the shock absorber is provided, the circuit board and the sensor substrate can be stably supported in the case unit, and occurrence of rattling or the like of the components can be suppressed.

In the aspect of the invention, the case unit may include a top case and a bottom case, the sensor unit may include a light receiving portion, a light emitting portion, and a light transmitting member that transmits light emitted from the light emitting portion and light incident to the light receiving portion, and the bottom case and the light transmitting member may be insert-molded.

With such a configuration, the bottom case formed of a non-light transmitting material and the light transmitting member formed of a light transmitting material can be formed using a simple manufacturing process with a small amount of man-hours, and thus, the biometric information detecting apparatus can be provided at low cost.

In another aspect of the invention relates to a biometric information detecting apparatus including: a band unit; a case unit that is attached to the band unit; a sensor unit that is provided in the case unit; and a processing unit that is provided in the case unit and detects biometric information based on a detection signal from the sensor unit, in which the case unit includes a top case and a bottom case, and the band unit and the top case are insert-molded.

According to this aspect of the invention, the sensor unit is provided in the case unit, and biometric information is detected based on a detection signal from the sensor unit. Further, since the insert-molding method is used as the method for forming the top case and the bottom case, the band unit and the top case can be formed of optimal materials, respectively. Further, during insert-molding, a process of adhering and attaching the top case to the band unit is not necessary, and thus, the manufacturing process can be simplified, and the biometric information detecting apparatus can be provided at low cost.

Still another aspect of the invention relates to a biometric information detecting apparatus including: a band unit; a case unit that is attached to the band unit; a sensor unit that is provided in the case unit; and a processing unit that is provided in the case unit and detects biometric information based on a detection signal from the sensor unit, in which a secondary battery and a circuit board on which the processing unit is mounted are provided in the case unit, the case unit includes a top case and a bottom case, the secondary battery is provided between a rear surface of the top case and the circuit board, and the rear surface of the top case is a curved surface.

According to this aspect of the invention, the sensor unit is provided in the case unit, and biometric information is detected based on a detection signal from the sensor unit. Further, if the secondary battery is disposed between the circuit board and the top case, the empty space of the circuit board on the side of the top case can be effectively used for disposition of the secondary battery, and thus, the case unit can be made to be thin and small, for example.

### BRIEF DESCRIPTION OF THE DRAWINGS

The invention will be described with reference to the accompanying drawings, wherein like numbers reference like elements.
Figs. 1A and 1B are diagrams illustrating an appearance of a biometric information detecting apparatus of an embodiment of the invention.
Fig. 2 is a diagram illustrating the appearance of the biometric information detecting apparatus of the present embodiment.
Fig. 3 is a diagram illustrating mounting of the biometric information detecting apparatus and communication with a terminal apparatus.
Fig. 4 is a functional block diagram of the biometric information detecting apparatus.
Figs. 5A and 5B are diagrams illustrating a sensor unit.
Fig. 6 is a diagram illustrating the relationship between a band unit and a case unit.
Fig. 7 is an exploded view illustrating an internal structure of the case unit, and the like.
Fig. 8 is an exploded view illustrating the internal structure of the case unit, and the like.
Fig. 9 is a cross-sectional view schematically illustrating the biometric information detecting apparatus.
Fig. 10 is a diagram illustrating an arrangement method of a secondary battery.

### DESCRIPTION OF EXEMPLARY EMBODIMENTS

Hereinafter, embodiments will be described with reference to the accompanying drawings. The embodiments to be described herein do not improperly limit the content of the invention disclosed in the appended claims. Further, all of the components described in the embodiments are not necessarily essential components of the invention.

### 1. Biometric information detecting apparatus

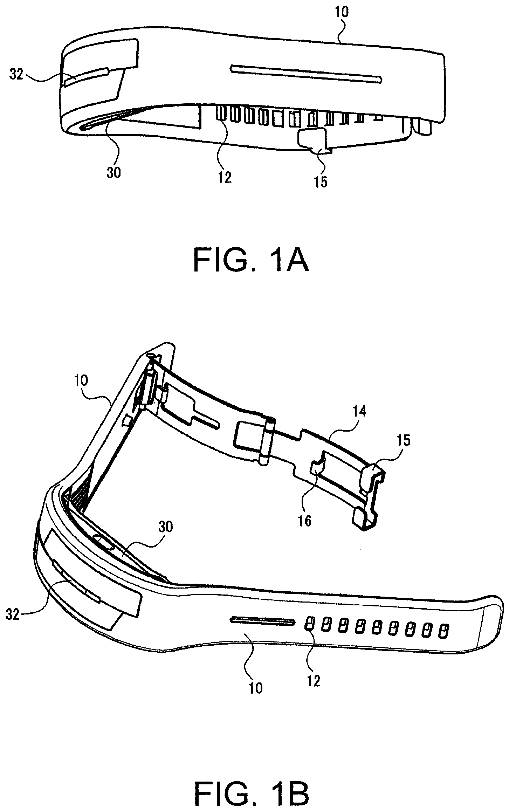

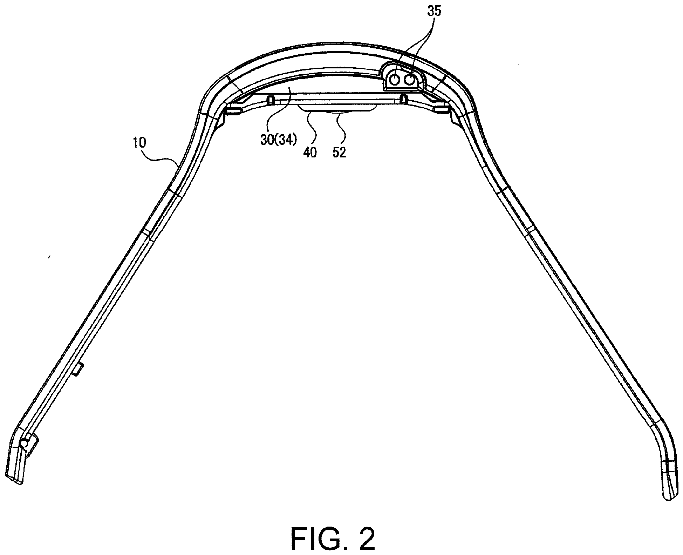

Figs. 1A and 1B, and Fig. 2 are diagrams illustrating an appearance of a biometric information detecting apparatus (biometric information measurement apparatus) according to an embodiment of the invention. Fig. 1A is a diagram illustrating the biometric information detecting apparatus when seen from a front surface direction, Fig. 1B is a diagram illustrating the biometric information detecting apparatus when seen from an upper direction, and Fig. 2 is a diagram illustrating the biometric information detecting apparatus when seen from a side surface direction.

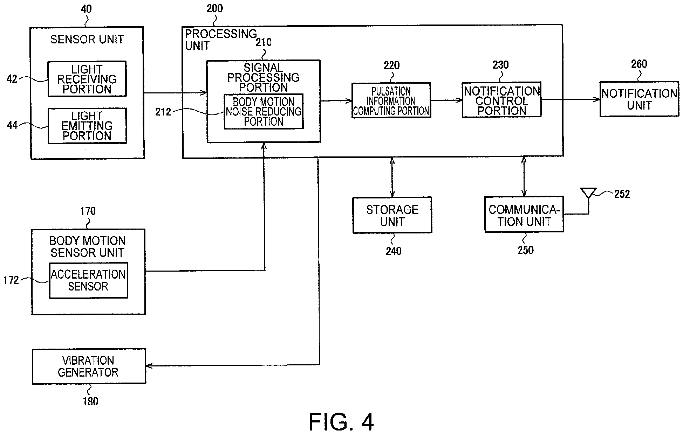

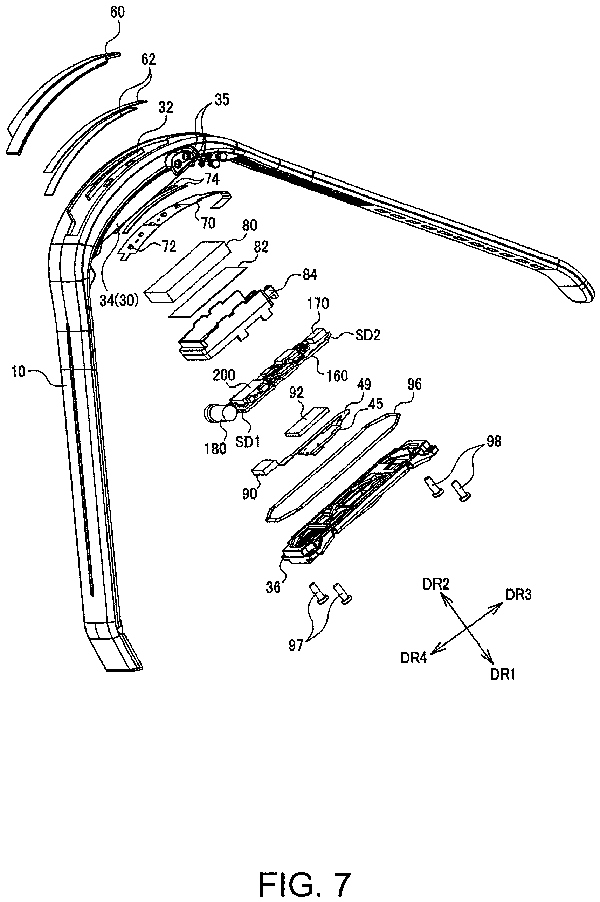

As shown in Figs. 1A and 1B, and Fig. 2, the biometric information detecting apparatus of the present embodiment includes a band unit 10, a case unit 30, and a sensor unit 40. The case unit 30 is attached to the band unit 10. The sensor unit 40 is provided in the case unit 30. Further, the biometric information detecting apparatus includes a processing unit 200, as shown in Figs. 4 and 7 to be described later. The processing unit 200 is provided in the case unit 30 to detect biometric information based on a detection signal from the sensor unit 40. The biometric information detecting apparatus of the present embodiment is not limited to the configuration of Figs. 1A and 1B, and Fig. 2, and various modifications such as omission of a part of the components, replacement of a part of the components with other components, or addition of other components are possible.

The band unit 10 is wound around the wrist of a user for installation of the biometric information detecting apparatus. The band unit 10 is formed with band holes 12, and a buckle portion 14. The buckle portion 14 includes a band inserting portion 15 and a protruding portion 16. The user inserts an end side of the band unit 10 into the band inserting portion 15 of the buckle portion 14 and inserts the protruding portion 16 of the buckle portion 14 into one of the band holes 12 of the band unit 10, to thereby mount the biometric information detecting apparatus on the wrist. In this case, accordingly, as the protruding portion 16 is inserted into the band holes 12, the magnitude of a pressing force of the sensor unit 40 to be described later (pressing force against a surface of the wrist) is adjusted.

The case unit 30 corresponds to a main body unit of the biometric information detecting apparatus. Inside the case unit 30, various components of the biometric information detecting apparatus such as the sensor unit 40 and the processing unit 200 are provided. That is, the case unit 30 is a housing that accommodates the components. The case unit 30 includes a top case 34 and a bottom case 36, as shown in Fig. 7 (to be described later). The case unit 30 may be configured so that the top case 34 and the bottom case 36 are not separated.

A light emitting window portion 32 is provided in the case unit 30. The light emitting window portion 32 is formed of a light emitting member. Further, as shown in Fig. 7 to be described later, the case unit 30 is provided with a light emitting portion 72 (LED) mounted to a flexible substrate 70. Light from the light emitting portion 72 is output to the outside of the case unit 30 through the light emitting window portion 32.

As shown in Fig. 2, the case unit 30 is provided with a terminal portion 35. If the biometric information detecting apparatus is mounted to a cradle (not shown), a terminal portion of the cradle and the terminal portion 35 of the case unit 30 are electrically connected to each other. Thus, a secondary battery 80 (battery) provided in the case unit 30 may be charged.

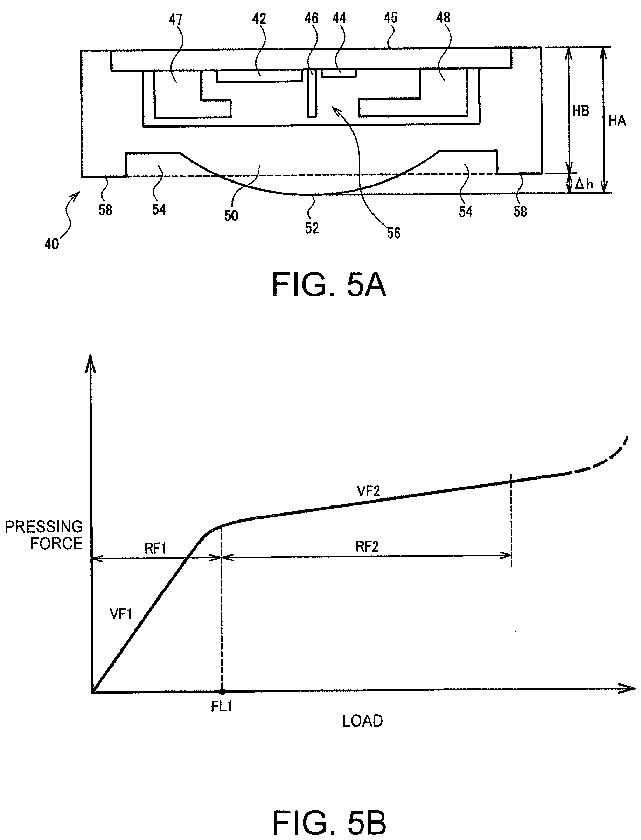

The sensor unit 40 detects biometric information such as a pulse wave of a test object. For example, the sensor unit 40 includes a light receiving portion 42 and a light emitting portion 44, as shown in Figs. 4 and 5A to be described later. Further, the sensor unit 40 includes a convex portion 52 that is formed of a light transmitting member and applies a pressing force when being in contact with the skin of the test object. In a state where the convex portion 52 applies the pressing force to the skin of the test object in this way, the light emitting portion 44 emits light, the light receiving portion 42 receives light obtained as the light is reflected from the test object (a blood vessel), and a light reception result is output to the processing unit 200 as a detection signal. In addition, the processing unit 200 detects the biometric information such as a pulse wave based on the detection signal from the sensor unit 40. The biometric information that is a detection target of the biometric information detecting apparatus of the present embodiment is not limited to the pulse wave (pulse rate). The biometric information detecting apparatus may be an apparatus that detects biometric information other than the pulse wave (for example, oxygen saturation in blood, body temperature, heart beats, or the like).

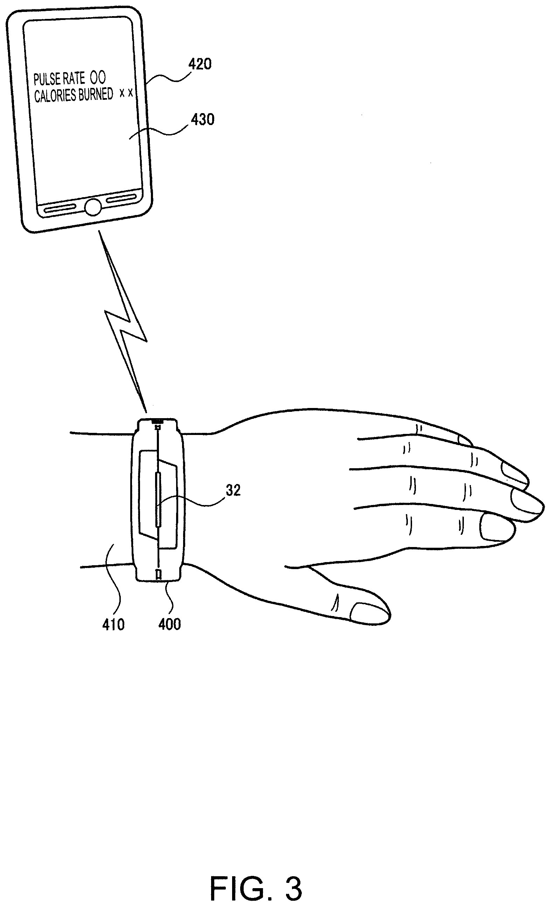

Fig. 3 is a diagram illustrating the mounting of a biometric information detecting apparatus 400, and communication thereof with a terminal apparatus 420.

As shown in Fig. 3, a user who is a test object wears the biometric information detecting apparatus 400 on the wrist 410 like a watch. As shown in Fig. 2, the sensor unit 40 is provided on the surface of the case unit 30 on the side of the test object. Accordingly, if the biometric information detecting apparatus 400 is mounted, the convex portion 52 of the sensor unit 40 comes into contact with the skin of the wrist 410 to apply the pressing force thereto, and in this state, the light emitting portion 44 of the sensor unit 40 emits light, and the light receiving portion 42 receives the reflected light, and thus, the biometric information such as a pulse wave is detected.

The biometric information detecting apparatus 400 and the terminal apparatus 420 are connected for communication, to thereby enable data exchange. The terminal apparatus 420 is a portable communication terminal such as a smart phone, a portable phone, or a future phone. Alternatively, the terminal apparatus 420 may be an information processing terminal such as a tablet computer. As the communication connection between the biometric information detecting apparatus 400 and the terminal apparatus 420, for example, proximity wireless communication such as Bluetooth (registered trademark) may be employed. In this way, as the biometric information detecting apparatus 400 and the terminal apparatus 420 are connected for communication, a variety of information such as a pulse rate or calories burned may be displayed on a display portion 430 (LCD or the like) of the terminal apparatus 420. That is, a variety of information obtained based on the detection signal of the sensor unit 40 may be displayed. Arithmetic processing of the information such as a pulse rate or calories burned may be executed in the biometric information detecting apparatus 400, or at least a part thereof may be executed in the terminal apparatus 420.

The light emitting window portion 32 is provided in the biometric information detecting apparatus 400, and a variety of information is notified to the user by light emission (lighting or flickering) of the light emitting portion 72 in FIG. 7. For example, when entering a fat combustion zone or when exiting from the fat combustion zone, this is notified by light emission of the light emitting portion 72 through the light emitting window portion 32. Further, if a mail or the like is received in the terminal apparatus 420, this is notified to the biometric information detecting apparatus 400 from the terminal apparatus 420. In addition, the light emitting portion 72 of the biometric information detecting apparatus 400 emits light, and thus, reception of the mail or the like is notified to the user.

In this way, in Fig. 3, the biometric information detecting apparatus 400 is not provided with a display portion such as an LCD, and information to be notified using characters, numbers or the like is displayed on the display portion 430 of the terminal apparatus 420. In this way, in Fig. 3, since the display portion such as an LCD is not provided and the minimum necessary information is notified to the user by light emission of the light emitting portion 72, reduction of the size of the biometric information detecting apparatus 400 is realized. Further, since the display portion is not provided in the biometric information detecting apparatus 400, a pleasant appearance of the biometric information detecting apparatus 400 can be achieved.

Fig. 4 is a functional block diagram of the biometric information detecting apparatus of the present embodiment. In Fig. 4, the biometric information detecting apparatus includes the sensor unit 40, a body motion sensor unit 170, a vibration generator 180, the processing unit 200, a storage unit 240, a communication unit 250, an antenna 252, and a notification unit 260. The biometric information detecting apparatus of the present embodiment is not limited to the configuration in Fig. 4, and various modifications such as omission of a part of the components, replacement of a part of the components with other components, or addition of other components is possible.

The sensor unit 40 detects the biometric information such as a pulse wave, and includes the light receiving portion 42 and the light emitting portion 44. A pulse wave sensor (photoelectric sensor) is realized by the light receiving portion 42, the light emitting portion 44, and the like. The sensor unit 40 outputs a signal detected by the pulse wave sensor as a pulse wave detection signal.

The body motion sensor unit 170 outputs a body motion detection signal that varies according to a body motion based on sensor information from various sensors. The body motion sensor unit 170 includes an acceleration sensor 172, for example, as the body motion sensor. The body motion sensor unit 170 may include a pressure sensor, a gyro sensor or the like as the body motion sensor.

The processing unit 200 performs a variety of signal processing or control processing tasks using the storage unit 240 as a work area, for example. For example, the processing unit 200 may be realized by a processor such as a CPU, or by a logic circuit such as an ASIC. The processing unit 200 includes a signal processing unit 210, a pulsation information computing portion 220, and a notification control portion 230.

The signal processing unit 210 performs a variety of signal processing tasks (filter processing or the like), for example, performs signal processing for the pulse wave detection signal from the sensor unit 40, the body motion detection signal from the body motion sensor unit 170, or the like. For example, the signal processing unit 210 includes a body motion noise reducing portion 212. The body motion noise reducing portion 212 performs a process of reducing (removing) body motion noise that is noise due to the body motion from the pulse wave detection signal based on the body motion detection signal from the body motion sensor unit 170. Specifically, for example, noise reduction processing using an adoptive filter or the like is performed.

The pulsation information computing portion 220 performs computation processing of pulsation information based on the signal from the signal processing unit 210 or the like. The pulsation information refers to information on the pulse rate or the like, for example. Specifically, the pulsation information computing portion 220 performs frequency analysis processing such as FFT with respect to the pulse wave detection signal following the noise reduction processing in the body motion noise reducing portion 212 to obtain a spectrum, and performs a process of setting a representative frequency in the obtained spectrum as a heart beat frequency. A value obtained by increasing the calculated frequency by 60 times is set as a generally used pulse rate (heartbeat rate). The pulsation information is not limited to the pulse rate, and for example, may be a variety of information indicating the pulse rate (frequency, cycle or the like of heart beat). Further, the pulsation information may be information indicating the state of pulsation. For example, a value indicating the blood volume may be used as the pulsation information.

The notification control portion 230 controls the notification unit 260. The notification unit 260 (notification device) notifies a variety of information to the user under the control of the notification control portion 230. As the notification unit 260, for example, the light emitting portion 72 such as an LED of Fig. 7 may be used. In this case, the notification control portion 230 controls the electric current that flows in the LED to control lighting, flickering or the like of the light emitting portion 72. The notification unit 260 may be a display such as an LCD, a buzzer, or the like.

Further, the notification control portion 230 controls the vibration generator 180. The vibration generator 180 notifies a variety of information to the user through vibration. The vibration generator 180 may be realized by a vibrating motor (vibrator), for example. The vibrating motor generates vibration by rotating eccentric spindles, for example. Specifically, the eccentric spindles are attached to both ends of a driving shaft (rotor shaft) so that the motor is vibrated. The vibration of the vibration generator 180 is controlled by the notification control portion 230. The vibration generator 180 is not limited to the vibrating motor, and various modifications may be used. For example, the vibration generator 180 may be realized by a piezoelectric element or the like, for example.

For example, notification of start-up when power is turned on, notification of initial success in pulse wave detection, and warning when a state where the pulse wave cannot be detected is continued for a predetermined time, and notification when the fat combustion zone changes, warning when a battery voltage is reduced, notification of a wake up alarm, notification of mail or a phone call from a terminal apparatus such as a smart phone, or the like may be performed through the vibration of the vibration generator 180. The information may be notified by the light emitting portion 720, or may be notified by both the vibration generator 180 and the light emitting portion 720.

The communication unit 250 performs communication with the outside terminal apparatus 420 described in Fig. 3. For example, the communication unit 250 performs wireless communication based on a standard such as Bluetooth (registered trademark), for example. Specifically, the communication unit 250 performs reception of a signal from the antenna 252 or transmission of a signal to the antenna 252. The function of the communication unit 250 may be realized by a communication processor or a logic circuit such as an ASIC.

### 2. Sensor unit

Fig. 5A shows a detailed configuration example of the sensor unit 40. The sensor unit 40 includes the light receiving portion 42 and the light emitting portion 44. The light receiving portion 42 and the light emitting portion 44 are mounted on a sensor substrate 45. The light emitting portion 42 receives light (reflected light, transmitted light or the like) from the test object. The light emitting portion 44 outputs light to the test object. For example, when the light emitting portion 44 outputs light to the test object and the light is reflected from the test object (blood vessel), the light receiving portion 42 receives the reflected light for detection. The light receiving portion 42 may be realized by a light receiving element such as a photodiode, for example. The light emitting portion 44 may be realized by a light emitting element such as an LED, for example. Also, the light receiving portion 42 may be realized by a PN junction diode element or the like formed on a semiconductor substrate. In this case, an angle limiting filter that narrows a light reception angle or a wavelength limiting filter that limits a wavelength of light that enters the light receiving element may be formed on the diode element.

For example, regarding a pulsimeter, light from the light emitting portion 44 travels inside the test object, and is diffused or scattered through the outer layer of the skin, the true skin, the subcutaneous tissue and the like. Then, the light reaches the blood vessel (portion to be detected), and is then reflected. Here, part of the light is absorbed by the blood vessel. Further, the rate of absorption of the light in the blood vessel varies due to the influence of pulsation and the light intensity of the reflected light also varies. Thus, the light receiving portion 42 is configured to receive the reflected light to detect the variation of the light intensity, thereby detecting the pulse rate or the like which is the biometric information.

A light shielding portion 46 (light shielding wall) is provided between the light receiving portion 42 and the light emitting portion 44. The light shielding portion 46 shields the light from the light emitting portion 44 from directly entering the light receiving portion 42, for example.

Further, stop portions 47 and 48 are provided in the sensor unit 40. The stop portions 47 and 48 narrow the light from the test object or the light from the light emitting portion 44 on an optical path between the test object and the sensor unit 40. In Fig. 5A, the stop portions 47 and 48 are provided between the light transmitting member 50 and the sensor unit 40. Here, the stop portions 47 and 48 may be provided between the light transmitting member 50 and the test object or inside the light transmitting member 50. Further, the light shielding portion 46 and the stop portions 47 and 48 may be integrally formed by sheet metal working, for example.

The light transmitting member 50 is provided on the surface of the biometric information detecting apparatus on the side that comes into contact with the test object, and transmits the light from the test object. Further, the light transmitting member 50 comes into contact with the test object when the biometric information of the test object is measured. For example, the convex portion 52 (detecting window) of the light transmitting member 50 comes into contact with the test object. It is preferable that the surface shape of the convex portion 52 is a curved surface shape (spherical shape), but the shape is not limited thereto, and various shapes may be used. Further, since it is sufficient if the light transmitting member 50 is transparent with respect to the wavelength of the light from the test object, the light transmitting member 50 may be formed of a colorless material, or may be formed of a colored material.

A recess portion 54 for suppressing variation of the pressing force or the like is provided around the convex portion 52 of the light transmitting member 50. Further, when the surface of the light transmitting member 50 on the side where the convex portion 52 is provided is referred to as a first surface, the light transmitting member 50 has the concave portion 56 at a position corresponding to the convex portion 52 on a second surface that is a rear side of the first surface. The light receiving portion 42, the light emitting portion 44, the light shielding portion 46, and the stop portions 47 and 48 are provided in the space of the concave portion 56.

In addition, on the surface of the biometric information detecting apparatus on the side of the test object, a pressing force suppressing portion 58 that suppresses the pressing force applied to the test object (skin of the wrist) by the convex portion 52 is provided. In Fig. 5A, the pressing force suppressing portion 58 is provided to surround the convex portion 52 of the light transmitting member 50.

Further, in Fig. 5A, when the height of the convex portion 52 in a direction orthogonal to the surface of the biometric information detecting apparatus on the side of the test object is HA (for example, the height of a top point of the convex portion 52 having a curved shape), the height of the pressing force suppressing portion 58 is HB (for example, the height in a highest place), and a value obtained by subtracting the height HB from the height HA (difference between the heights HA and HB) is Δh, the relationship of Δh=HA-HB>0 is established. For example, the convex portion 52 protrudes from the pressing force suppressing portion 58 toward the test object so that Δh>0. That is, the convex portion 52 protrudes toward the test object by Δh from the pressing force suppressing portion (pressing force suppressing surface) 58.

In this way, as the convex portion 52 where Δh>0 is provided, an initial pressing force exceeding, for example, a vein vanishing point may be applied to the test object. Further, as the pressing force suppressing portion 58 for suppressing the pressing force applied to the test object by the convex portion 52 is provided, the pressing force variation may be suppressed to the minimum in a usage range where the measurement of the biometric information is performed by the biometric information detecting apparatus, to thereby achieve reduction of a noise component or the like. In addition, if the convex portion 52 protrudes from the pressing force suppressing portion 58 so that Δh>0, after the convex portion 52 comes into contact with the test object to apply the initial pressing force, the pressing force suppressing portion 58 comes into contact with the test object, so that the pressing force applied to the test object by the convex portion 52 can be suppressed. Here, the vein vanishing point refers to a point where a signal due to the vein overlaps with the pulse wave signal, when the convex portion 52 is in contact with the test object and the pressing force becomes gradually stronger, vanishes or becomes smaller to the degree of having no influence on the pulse wave measurement.

For example, in Fig. 5B, a transverse axis represents a load generated by a load mechanism (mechanism configured by the band unit, the buckle portion or the like) of the biometric information detecting apparatus, and a longitudinal axis represents the pressing force (pressure applied to the blood vessel) applied to the test object by the convex portion 52. Further, the amount of variation of the pressing force of the convex portion 52 with respect to the load due to the load mechanism for generating the pressing force of the convex portion 52 is referred to as a pressing force variation amount. The pressing force variation amount corresponds to a slope of a variation characteristic of the pressing force with respect to the load.

In this case, the pressing force suppressing portion 58 suppress the pressing force applied to the test object with the convex portion 52 so that a pressing force variation amount VF2 in a second load range RF2 where the load of the load mechanism which becomes larger than FL1 then becomes small, compared with a pressing force variation amount VF1 in a first load range RF1 where the load of the load mechanism becomes 0 to FL1. That is, in the first load range RF1 that is an initial pressing force range, the pressing force variation amount VF1 is set to be large, but in the second load range RF2 that is a usage range of the biometric information detecting apparatus, the pressing force variation amount VF2 is set to be small.

That is, in the first load range RF1, the pressing force variation amount VF1 is set to be large, and the slope of the variation characteristic of the pressing force with respect to the load is set to be large. The pressing force having such a large slope of the variation characteristic is realized by Δh corresponding to the projecting amount of the convex portion 52. That is, by providing the convex portion 52 where Δh>0, even when the load due to the load mechanism is small, the initial pressing force necessary and sufficient for exceeding the vein vanishing point may be applied to the test object.

In contrast, in the second load range RF2, the pressing force variation amount VF2 is set to be small, and the slope of the variation characteristic of the pressing force with respect to the load is set to be small. The pressing force having such a small slope of the variation characteristic is realized by suppressing the pressing force using the pressing force suppressing portion 58. That is, by suppressing the pressing force applied to the test object by the convex portion 52 by the pressing force suppressing force 58, even when there is variation of the load or the like in the usage range of the biometric information detecting apparatus, the pressing force variation can be suppressed to the minimum. Thus, reduction of a noise component or the like is achieved.

In this way, by applying the optimized pressing force (for example, about 16 kPa) to the test object, a pulse wave detection signal having a higher M/N ratio (S/N ratio) can be obtained. That is, a signal component of the pulse wave sensor can be increased, and a noise component thereof can be reduced. Here, M represents a signal level of the pulse wave detection signal, and N represents the noise level thereof.

In addition, by setting the range of the pressing force used for measurement of the pulse wave to a range corresponding to the second load range RF2, the pressing force variation can be suppressed to the minimum (for example, about ±4 kPa), and thus, the noise component can be reduced.

### 3. Detailed structure example

### 3.1 Band unit and case unit

The biometric information detecting apparatus of the present embodiment as described above includes the band unit 10, the case unit 30 attached to the band unit 10, the sensor unit 40 provided to the case unit 30, and the processing unit 200 that is provided in the case unit 30 and detects the biometric information based on the detection signal from the sensor unit 40.

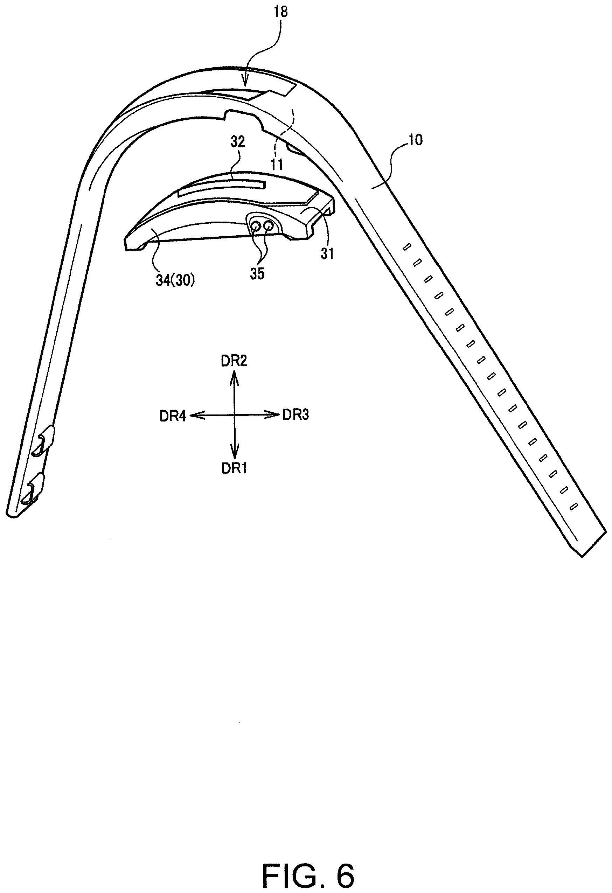

Further, as shown in Fig. 6, the band unit 10 has an inner surface 11 of a first curved surface shape that faces the test object when the biometric information detecting apparatus is mounted, and the case unit 30 (top case 34) has an outer surface 31 of a second curved surface shape that faces the inner surface 11 of the band unit 10.

For example, in Fig. 6, a downward direction DR1 is an inward direction toward the side of the test object, an upward direction DR2 is an outward direction toward the outside of the test object. The inner surface 11 of the band unit 10 is a surface directed along the direction DR1 that is the inward direction, and the outer surface 31 of the case unit 30 is a surface directed along the direction DR2 that is the outward direction. Both the inner surface 11 of the band unit 10 and the outer surface 31 of the case unit 30 are formed in curved shapes having the same curvature (curved surface shapes having approximately the same curvatures), for example. That is, the inner surface 11 of the central part of the hand portion 10 is formed in the first curved surface shape having a curvature based on the curvature of the wrist so as to fit the wrist when the band unit 10 is attached to the wrist. Further, the outer surface 31 of the case unit 30 that faces and comes into close contact with the inner surface 11 is formed in the second curved surface shape having a curvature corresponding to the curvature of the first curved surface shape of the inner surface 11.

With such a configuration as shown in Fig. 6, when the biometric information detecting apparatus is attached to the wrist, the thickness of the case unit 30 in the direction DR1 can be made to be thin, and thus, the volume of the case unit 30 can be made to be small. Accordingly, reduction of the size and weight of the biometric information detecting apparatus can be achieved, and thus, the burden of the user during long-time wearing of the biometric information detecting apparatus can be reduced. Further, in this way, by reducing the burden of the user during the long-time wearing of the apparatus, the biometric information can be continuously measured, and the biometric information detecting apparatus suitable for performing life-log of the biometric information can be provided.

That is, in order to perform the life-log of the biometric information, it is necessary that the user constantly wears the biometric information detecting apparatus, but if the biometric information detecting apparatus is large or the weight thereof is heavy, the burden of the user when wearing the biometric information detecting apparatus increases, which may interrupt the life-log measurement of the biometric information based on the constant wearing of the apparatus.

In this regard, in Fig. 6, the inner surface 11 of the band unit 10 and the outer surface 31 of the case unit 30 are formed having curved surface shapes of the same curvature, for example, and thus, the volume of the case unit 30 can be minimized and the thickness in the direction DR1 can be made to be thin. Thus, a small and light biometric information detecting apparatus can be easily realized, and thus, a biometric information detecting apparatus suitable for the life-log measurement can be realized.

In addition, when the user normally uses the biometric information detecting apparatus, the design (appearance) of the biometric information detecting apparatus is an important factor. In this regard, by using the structure as shown in Fig. 6, a biometric information detecting apparatus giving a simple, compact and stylish impression can be realized. Accordingly, motivation or the like can be increased when the user constantly wears the biometric information detecting apparatus, and a biometric information detecting apparatus suitable for the life-log measurement can be realized.

The first curved surface shape of the inner surface 11 of the band unit 10 and the second curved surface shape of the outer surface 31 of the case unit 30 may be the same curved surface shape, or may be different curved surface shapes. For example, the curvature of the first curved surface shape and the curvature of the second curved surface shape may be the same, or may be different from each other. For example, if the first curved surface shape and the second curved surface shape are the same curved surface shape (for example, the curved surface shape having the same curvature), since the band unit 10 and the case unit 30 are fixed without any gap between them, there is an advantage in that deviation hardly occurs therebetween. On the other hand, if the first curved surface shape and the second curved surface shape are different curved shapes (for example, curved surface shapes having different curvatures), the degree of freedom in design can be enhanced.

Further, in the present embodiment, the case unit 30 includes the top case 34 and the bottom case 36, in which at least the top case 34 and the band unit 10 are insert-molded. For example, the top case 34 (at least a part of the case unit 30) and the band unit 10 are integrally formed using insert-molding.

By performing the insert-molding, members of different materials may be integrally formed. For example, the band unit 10 may be formed of a first material, and the top case 34 (case unit 30) may be formed of a second material harder than the first material. That is, in order to provide a good fit on the wrist when wearing the biometric information detecting apparatus, for example, the band unit 10 may be formed of the first material such as soft silicon resin. On the other hand, in order to suppress breakage, failure or the like of the built-in components, the top case 34 may be formed of the second material such as hard plastic. The materials that form the band unit 10 and the top case 34 are not limited thereto, and various modifications may be used. For example, the band unit 10 maybe formed of a soft material (for example, urethane) other than the silicon resin, and the top case 34 (case unit 30) may be formed of a material (for example, metal) other than the plastic (polycarbonate).

By performing insert-molding, as described above, the band unit 10 and the top case 34 of different materials can be easily and integrally formed. For example, by performing insert-molding in which the silicon resin or the like for forming the band unit 10 is poured into the top case 34 set in a mold (mold for integral formation), such an integral formation can be achieved.

If the above-described insert-molding method is employed, the band unit 10 and the top case 34 can be formed of preferable materials, respectively. For example, by forming the band unit 10 of a soft material, a good fit can be provided when wearing the biometric information detecting apparatus on the wrist. Further, by forming the top case 34 or the bottom case 36 of a hard material, breakage, failure or the like of the built-in components in the case unit 30 can be suppressed. In addition, during insert-molding, a process of mounting and attaching the top case 34 to the band unit 10 is not necessary, and thus, the manufacturing process can be simplified. Thus, the biometric information detecting apparatus can be provided at low cost, for example.

Furthermore, as shown in Fig. 6, in a state where the band unit 10 and the top case 34 are integrally formed, a part of the outer surface 31 of the top case 34 is exposed in the outward direction DR2, and the light emitting window portion 32 is provided in the exposed part. The light from the light emitting portion 72 is output in the outward direction DR2 through the light emitting window portion 32 provided in the exposed part, so that a variety of information can be notified to the user.

According to such a configuration, the variety of information can be notified to the user using the light emitting portion 72 without providing a display unit such as an LCD. Further, since the display unit such as an LCD is not provided in the case unit 30, reduction of the size and weight of the biometric information detecting apparatus can be achieved, and a pleasant appearance can be achieved, and a biometric information detecting apparatus suitable for the life-log measurement can be realized.

Further, in the band unit 10 (inner surface of the band unit 10), a hole portion 18 that accommodates the top case 34 (at least a part of the case unit 30) is provided. Specifically, in the inner surface of the central part of the band part 10, the hole portion 18 having a shape corresponding to the shape of the outer surface of the top case 34 is formed.

With such a configuration, the band unit 10 and the top case 34 can be integrally formed in a state where the top case 34 is accommodated in the hole portion 18 so that the top case 34 is covered by the band unit 10. Further, in this way, by providing the hole portion 18 , the thickness in the direction DR1 can be suppressed to the minimum, and thus, reduction of the size of the biometric information detecting apparatus can be achieved, for example.

### 3.2 Internal structure of case unit

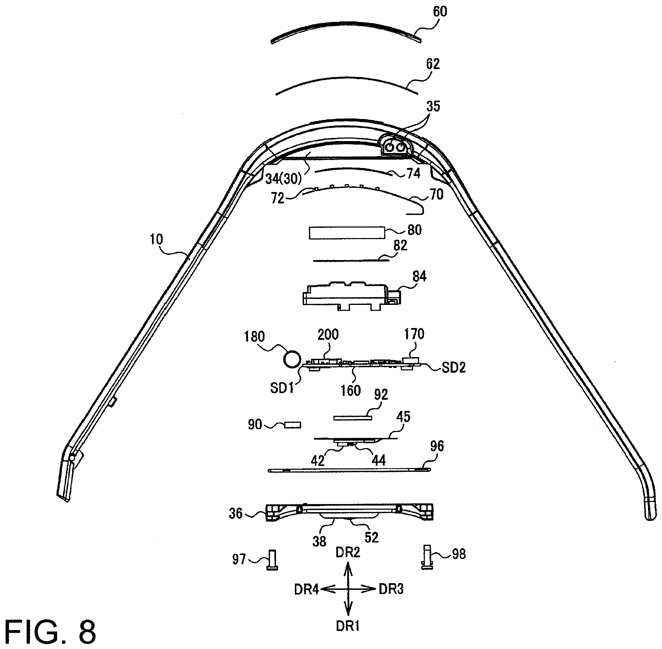

Figs. 7 and 8 are exploded views illustrating an internal structure of the case unit 30. Reference numeral 60 represents a decoration cover, and reference numeral 62 represents double sided tape. The decoration cover 60 is attached to the outer surface of the top case 34 by the double sided tape 62. By providing the decoration cover 60 with such a configuration, a pleasant appearance of the biometric information detecting apparatus can be achieved.

Reference numeral 70 represents a flexible substrate, and reference numeral 74 represents double sided tape. The light emitting portion 72 such as an LED is mounted on the flexible substrate 70. The antenna 252 shown in Fig. 4 is provided on the flexible substrate 70. Specifically, a metal pattern (not shown) of the antenna 252 is formed on the flexible substrate 70.

Reference numeral 80 represents a secondary battery (battery), reference numeral 82 represents double sided tape, and reference numeral 84 represents a holder of the secondary battery 80. The secondary battery 80 is attached to the holder 84 by the double sided tape.

Reference numeral 160 represents a circuit board (main board), reference numeral 170 represents the body motion sensor unit, reference numeral 180 represents the vibration generator (vibrating motor), and reference numeral 200 represents the processing unit. The body motion sensor unit 170 and the processing unit 200 are mounted on the circuit board 160.

Reference numeral 45 represents a sensor substrate, and reference numeral 49 represents a connection cable. As shown in Fig. 5A, the light receiving portion 42, the light emitting portion 44 and the like are mounted on the sensor substrate 45. The sensor substrate 45 and the circuit board 160 are electrically connected to each other by the connection cable 49. Reference numerals 90, 92 and 96 represent shock absorbers.

Reference numeral 36 represents the bottom case, and reference numerals 97 and 98 represent screws. The top case 34 and the bottom case 36 are fastened by the screws 97 and 98.

### 3.3 Light emitting portion and flexible substrate

As shown in Figs. 7 and 8, in the biometric information detecting apparatus of the present embodiment, on a rear surface side of the outer surface 31 of the case unit 30, the flexible substrate 70 on which the light emitting portion 72 is disposed is provided. Specifically, the flexible substrate 70 is provided on the side, in the DR1 direction, of the top case 34 that forms the case unit 30 (immediately under the top case 34). For example, the flexible substrate 70 is provided between the top case 34 and the secondary battery 80. The flexible substrate 70 is attached to the inner surface (rear surface of the outer surface 31) of the top case 34 by the double sided tape 74. Further, the plural light emitting portions 72 (LED) are mounted on the flexible substrate 70, and the light from the light emitting portion 72 is irradiated to the outside through the light emitting window portion 32.

According to such a configuration, a variety of information can be notified to the user by lighting, flickering or the like of the light of the light emitting portion 72. Further, since the light emitting portion 72 is mounted on the thin flexible substrate 70, the thickness of the case unit 30 can be made to be thin. In addition, as described with reference to Fig. 6, the outer surface 31 of the case unit 30 is formed in the curved surface shape corresponding to the curved surface of the inner surface 11 of the band unit 10, and the rear surface of the outer surface 31 is also formed in a curved surface shape. Accordingly, if the flexible substrate 70 is used, the flexible substrate 70 can be disposed to be bent in a curved surface shape to be fitted to the curved surface shape of the rear surface of the outer surface 31, and the light emitting portion 72 can be mounted on the flexible substrate 70.

Further, in Figs. 7 and 8, the top case 34 includes the light emitting window portion 32 for the light emitting portion 72, and the top case 34 and the light emitting window portion 32 are insert-molded. For example, the top case 34 and the light emitting window portion 32 are integrally formed using insert-molding. For example, by performing insert-molding so that light transmitting silicon resin or the like is poured into a portion of the top case 34 corresponding to the light emitting window portion 32, set in a mold, such an integral formation can be achieved.

In addition, for example, the light emitting window portion 32 is formed of a light transmitting material, and the top case 34 is formed of a non-light transmitting material. Specifically, the top case 34 (case unit 30) is formed of plastic (polycarbonate) colored black or the like, for example, and the light emitting window portion 32 is formed of light transmitting plastic (polycarbonate) or the like. Here, it is sufficient if the "light transmitting" is transparent with respect to the wavelength of the light from the test object, and thus, the "light transmitting" may include color (for example, milk-white). Furthermore, "non-light transmitting" means that the material does not transmit light of a wavelength detectable by the biometric information detecting apparatus.

In this way, if the top case 34 and the light emitting window portion 32 are integrally formed using insert-molding, the top case 34 formed of the non-light transmitting material and the light emitting window portion 32 formed of the light transmitting material can be integrally formed using a simple manufacturing process with a small amount of man-hours. Accordingly, the biometric information detecting apparatus can be provided at low cost, for example.

In the manufacturing process using insert-molding, first, the top case 34 and the light emitting window portion 32 are integrally formed using insert-molding. Then, the top case 34 and the band unit 10 in which the light emitting window portion 32 is insert-molded are integrally formed using insert-molding.

Further, on the flexible substrate 70 disposed on the rear surface side of the outer surface 31 of the case unit 30, the antenna 252 (see Fig. 4) for communication (proximity wireless communication) is provided. Specifically, the metal pattern that forms the antenna 252 is formed on the flexible substrate 70. In this case, as the metal pattern, for example, various patterns such as a ring shape or a zigzag shape may be used.

In this way, if the antenna 252 is formed on the flexible substrate 70, the antenna 252 can be disposed at a position close to the outer surface 31 of the case unit 30, and thus, reception sensitivity or the like of the antenna 252 can be enhanced. Further, the flexible substrate 70 can be commonly used for disposition of the light emitting portion 72 and formation of the antenna 252, and thus, the number of parts can be reduced and reduction of the size of the apparatus can be achieved.

### 3.4 Circuit board, secondary battery, vibration generator, and shock absorber

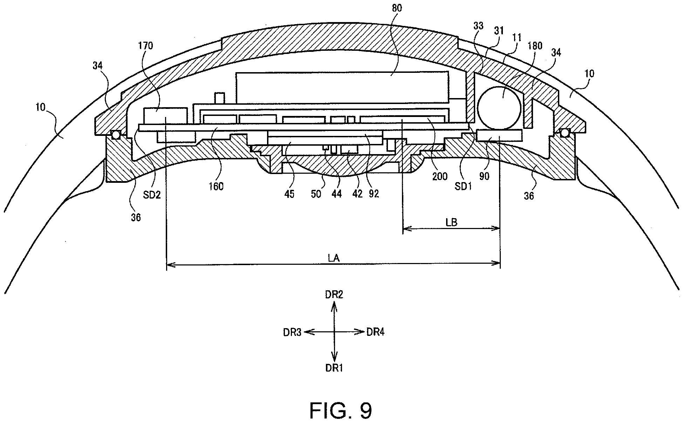

As shown in Figs. 7 and 8, the circuit board 160 on which the processing unit 200 is mounted is provided in the case unit 30. The circuit board 160 is a rigid substrate, for example. Further, as shown in Fig. 9 which is a schematic cross-sectional view of the biometric information detecting apparatus, the secondary battery 80 is disposed between the circuit board 160 and the top case 34 (outer surface of the case unit 30). Specifically, the secondary battery 80 is provided between the rear surface 33 of the top case 34 and the circuit board 160, and the rear surface 33 of the top case 34 is formed in the curved surface shape.

The secondary battery 80 supplies power to the circuit board 160 (the processing unit 200 and the body motion sensor unit 170), the vibration generator 180, the sensor unit 40, and the like. For example, by mounting the biometric information detecting apparatus to a cradle, a terminal portion of the cradle and the case terminal portion 35 are electrically connected to each other, and the secondary battery 80 is charged by the power from the cradle. As the secondary battery 80, for example, a lithium ion polymer battery or the like may be employed.

In the present embodiment, the secondary battery 80 is disposed between the circuit board 160 and the top case 34. Accordingly, an empty space on the side of the circuit board 160 in the DR2 direction can be effectively used for disposition of the secondary battery 80. For example, since the inner surface 33 of the top case 34 is formed in the curved surface shape, a relatively wide empty space can be secured on the side of the circuit board 160 in the DR2 direction. In the present embodiment, the secondary battery 80 with a large volume is disposed in the empty space. Thus, the component arrangement in which the space in the case unit 30 is effectively used can be achieved, and reduction of the thickness and size of the case unit 30 can be achieved, for example.

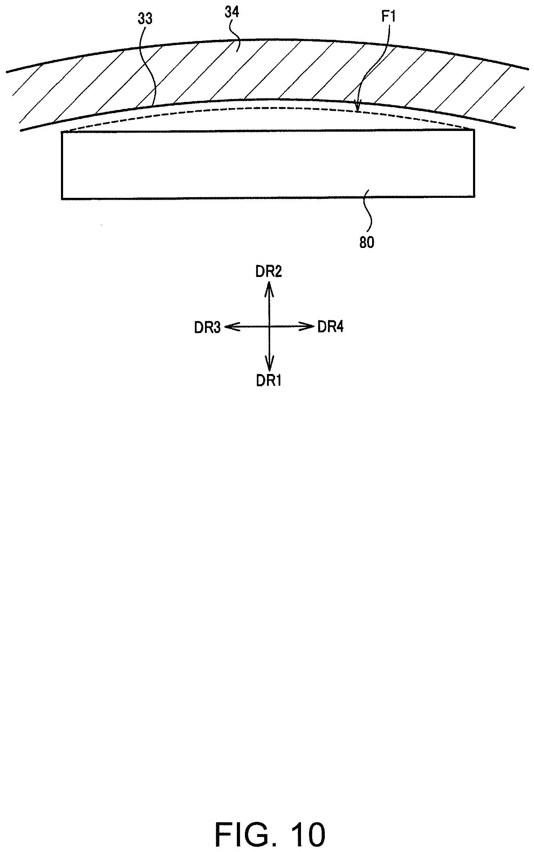

Further, in the secondary battery 80, as the charging or the like is repeated, the central part thereof may be expanded as indicated by F1 in Fig. 10. That is, the central part is expanded in the DR2 direction. In addition, as described above, the thickness of the case unit 30 is suppressed to the minimum, but if such expansion of the secondary battery 80 due to the charging occurs, a clearance between the components may not be secured.

In this regard, in the present embodiment, the secondary battery 80 is provided between the rear surface 33 of the top case 34 and the circuit board 160, and as shown in Fig. 9, the rear surface 33 of the top case 34 is formed in the curved surface shape. Accordingly, the empty space on the side of the secondary battery 80 in the DR2 direction can be secured. For example, since a space of about 1 mm (about 30% in battery volume ratio) can be secured in the central part, even when the central part of the secondary battery 80 is expanded in the DR2 direction, this problem can be handled.

Further, in the present embodiment, the circuit board 160 on which the processing unit 200 is mounted and the vibration generator 180 are provided in the case unit 30. Furthermore, as shown in Figs. 7 and 8, the vibration generator 180 is provided on a first side SD1 among the first side SD1 and a second side SD2 of the circuit board 160 that are opposite to each other. Specifically, the vibration generator 180 is disposed in the vicinity of the first side SD1 of the circuit board 160. The first and second sides SD1 and SD2 are sides corresponding to short sides of the circuit board 160, for example. For example, since it is considered that a space is easily secured on the short sides of the circuit board 160 in view of its structure, if the vibration generator 180 is disposed on the first side SD1 that is a short side of the circuit board 160, the empty space can be effectively used. However, the first side SD1 on which the vibration generator 180 is disposed may be a long side of the circuit board 160.

In this way, if the vibration generator 180 is disposed, an empty space of an end portion of the circuit board 160 can be effectively used for disposition of the vibration generator 180. For example, in Fig. 9, the empty space is present in the end portion of the circuit board 160 in a DR4 direction (an end portion of the circuit board 160 on the side SD1), and the vibration generator 180 is disposed in the empty space. For example, the vibration generator 180 is disposed between the rear surface 33 of the top case 34 and the bottom case 36 in an end portion (right end portion) of the case unit 30 in the DR4 direction. A direction DR3 is a direction orthogonal to the direction DR1, and the direction DR4 is a direction opposite to the direction DR3.

In this way, as the vibration generator 180 having a relatively large volume is disposed in the empty space of the case unit 30 in the direction DR4, the component arrangement in which the space in the case unit 30 is effectively used can be achieved, and reduction in the thickness and size of the case unit 30 can be achieved, for example.

Furthermore, in the present embodiment, the body motion sensor unit 170 is mounted on the circuit board 160 in addition to the processing unit 200. By providing the body motion sensor unit 170, the process of reducing the body noise as described in Fig. 4 can be performed, for example.

In addition, as shown in Fig. 9, when the distance between the vibration generator 180 and the body motion sensor unit 170 is LA and the distance between the vibration generator 180 and the processing unit 200 is LB, the relationship of LA>LB is established. Here, the distance LA refers to the distance between a representative position (central position) of the vibration generator 180 and a representative position (central position) of the body motion sensor unit 170. The distance LB refers to the distance between the representative position (central position) of the vibration generator 180 and a representative position (central position) of the processing unit 200. For example, the vibration generator 180 and the processing unit 200 are disposed on the first side SD1 of the circuit board 160. On the other hand, the body motion sensor unit 170 is disposed on the second side SD2 of the circuit board 160. That is, the vibration generator 180 is disposed at a position more distant from the body motion sensor unit 170, than from the processing unit 200.

With such a configuration, the distance between the vibration generator 180 and the body motion sensor unit 170 can be made to be long, and the negative influence of vibration generated in the vibration generator 180 on the body motion sensor unit 170 can be reduced. For example, as described above, when the vibration generator 180 is disposed on the first side SD1 of the circuit board 160 to effectively use the space of the case unit 30, the body motion sensor 170 is provided on the second side SD2 opposite to the first side SD1. Accordingly, by increasing the distance between the vibration generator 180 and the body motion sensor unit 170 while achieving reduction in the size of the case unit 30 by appropriate arrangement of the vibration generator 180, the negative influence on the body motion sensor unit 170 due to the vibration generator 180 can be reduced.

Further, the vibration generator 180 is provided in the case unit 30, and the shock absorber 90 is provided between the vibration generator 180 and the bottom case 36. That is, the shock absorber 90 is provided on a side in the DR1 direction with reference to the vibration generator 180.

In this way, if the shock absorber 90 is provided, occurrence of a so-called chattering noise or the like when the vibration generator 180 is vibrated can be suppressed. Further, a variety of information can be notified to the user using appropriate vibration from which an uncomfortable vibration component or the like is removed. Further, as the shock absorber 90 is provided and the vibration generator 180 is provided thereon, the vibration generator 180 can be stably supported in the case unit 30, and occurrence of rattling or the like of the components can be suppressed.

Further, the circuit board 160 and the sensor substrate 45 on which the sensor unit 40 is mounted are provided in the case unit 30, and the shock absorber 92 is provided between the circuit board 160 and the sensor substrate 45.

In this way, as the shock absorber 92 is provided, the circuit board 160 or the sensor substrate 45 can be stably supported in the case unit 30, and occurrence of rattling or the like of parts can be suppressed. In addition, for example, when the sensor substrate 45 is press-fitted to be attached to the bottom case 36, since the shock absorber 92 is disposed above the sensor substrate 45, the attachment state due to the press-fitting can be stabilized. As the shock absorbers 90 and 92, for example, a member such as a urethane material may be employed.

Further, as described in Fig. 5A, the sensor unit 40 includes the light receiving portion 42, the light emitting portion 44, and the light transmitting member 50 that transmits light emitted from the light emitting portion 44 and light incident to the light receiving portion 42. The light transmitting member 50 is used as a cover of the sensor unit 40. In addition, in the present embodiment, the bottom case 36 and the light transmitting member 50 of the sensor unit 40 are also insert-molded. For example, the bottom case 36 and the light transmitting member 50 are integrally formed using insert-molding. For example, by performing insert-molding so that the material of the bottom case 36 is poured into the light transmitting member 50 (cover) set in a mold, such an integral formation can be achieved.

Furthermore, for example, the light transmitting member 50 is formed of a light transmitting material, and the bottom case 36 is formed of a non-light transmitting material. Specifically, the bottom case 36 is formed of non-light transmitting plastic (polycarbonate) or the like colored black, similar to the top case 34. In contrast, the light transmitting member 50 is formed of light transmitting plastic (polycarbonate).

In this way, if the bottom case 36 and the light transmitting member 50 of the sensor unit 40 are integrally formed using insert molding, the bottom case 36 formed of the non-light transmitting material and the light transmitting member 50 formed of the light transmitting material can be integrally formed using a simple manufacturing process with a small amount of man-hours.

Hereinabove, the embodiments of the invention have been described in detail, but it can be easily understood to those skilled in the art that various modifications can be made in a range without substantially departing from the novel contents and effects of the invention. Accordingly, such modifications should be construed to be included in the scope of the invention. For example, in the description and the drawings, a term written at least one time together with a different term having a wider meaning or the same meaning can be exchanged with the different term in any location in the description and the drawings. Further, the configuration and the operation of the biometric information detecting apparatus are not limited to the above-described embodiments, and various modifications may be realized within the scope of the claims.

## Claims

1. A biometric information detecting apparatus comprising:
a band unit (10);
a case unit (30) that is attached to the band unit (10) ;
a sensor unit (40) that is provided in the case unit (30) ; and
a processing unit (200) that is provided in the case unit (30) and detects biometric information based on a detection signal from the sensor unit (40), wherein
the band unit (10) includes an inner surface (11) having a first curved surface shape on a side thereof that faces a test object when wearing the biometric information detecting apparatus,
the case unit (30) includes an outer surface (31) having a second curved surface shape that faces the inner surface (11) of the band unit (10), and
a circuit board (160) on which the processing unit (200) is mounted and a sensor substrate (45) on which the sensor unit (40) is mounted are provided in the case unit (30),
**characterized in that**:
a shock absorber (92) is provided between the circuit board (160) and the sensor substrate (45).

2. The biometric information detecting apparatus according to claim 1, wherein
the case unit (30) includes a top case (34) and a bottom case (36), and
at least the top case (34) and the band unit (10) are insert-molded.

3. The biometric information detecting apparatus according to claim 2, wherein
the band unit (10) is formed of a first material, and the top case (34) is formed of a second material harder than the first material.

4. The biometric information detecting apparatus according to any of claims 1-3, wherein
the case unit (30) includes a top case (34) and a bottom case (36), and
a hole portion (18) that accommodates the top case (34) is provided in the band unit (10).

5. The biometric information detecting apparatus according to any of claims 1-4, wherein
a flexible substrate (70) on which a light emitting portion (72) is disposed is provided on a rear surface side of the outer surface (31) of the case unit (30).

6. The biometric information detecting apparatus according to any of claims 1-5, wherein
the case unit (30) includes a top case (34) and a bottom case (36),
the top case (34) includes a light emitting window portion for the light emitting portion, and
the top case and the light emitting window portion (32) are insert-molded.

7. The biometric information detecting apparatus according to any of claims 1-6, wherein
a flexible substrate (70) on which a communication antenna is disposed is provided on a rear surface of the outer surface of the case unit (30).

8. The biometric information detecting apparatus according to any of claims 1-7, wherein
the case unit (30) includes a top case (34) and a bottom case (36), and
a battery (80) is disposed between the circuit board (160) and the top case (34).

9. The biometric information detecting apparatus according to any of claims 1-8, wherein
a vibration generator is provided in the case unit (30), and
the vibration generator (180) is provided, among a first side and a second side of the circuit board (160) that are opposite to each other, on the first side.

10. The biometric information detecting apparatus according to claim 9, wherein
a body motion sensor unit (170) is mounted on the circuit board (160), and
a distance between the vibration generator (180) and the body motion sensor unit (170) is longer than a distance between the vibration generator (180) and the processing unit (200).

11. The biometric information detecting apparatus according to claim 1, wherein
a vibration generator is provided in the case unit,
the case unit includes a top case and a bottom case, and
shock absorber is provided between the vibration generator and the bottom case.

12. The biometric information detecting apparatus according to any of claims 1-11, wherein
the case unit (30) includes a top case (34) and a bottom case (36),
the sensor unit (40) includes a light receiving portion (42), a light emitting portion (44), and a light transmitting member (50) that transmits light emitted from the light emitting portion (44) and light incident to the light receiving portion (42), and
the bottom case (36) and the light transmitting member (50) are insert-molded.

## Patentansprüche

1. Biometrische Datenerkennungsvorrichtung, umfassend:
eine Band-Einheit (10);
eine Gehäuse-Einheit (30), die an der Band-Einheit (10) befestigt ist;
eine Sensor-Einheit (40), die in der Gehäuse-Einheit (30) vorgesehen ist;
und
eine Verarbeitungs-Einheit (200), die in der Gehäuse-Einheit (30) vorgesehen ist und biometrische Daten, basierend auf einem Detektionssignal von der Sensor-Einheit (40), detektiert, wobei
die Band-Einheit (10) eine innere Oberfläche (11) aufweist mit einer ersten gekrümmten Oberflächen-Form auf einer Seite davon, die zu einem Prüfgegenstand weist, wenn die biometrische Datenerkennungsvorrichtung getragen wird,
die Gehäuse-Einheit (30) eine äußere Oberfläche (31) aufweist mit einer zweiten gekrümmten Oberflächen-Form, die zu der inneren Oberfläche (11) der Band-Einheit (10) weist, und
eine Schaltplatine (160), auf der die Verarbeitungs-Einheit (200) befestigt ist, und ein Sensor-Substrat (45), auf dem die Sensor-Einheit (40) befestigt ist, in der Gehäuse-Einheit (30) vorgesehen sind, **dadurch gekennzeichnet, dass**:
ein Stoßdämpfer (92) zwischen der Schaltplatine (160) und dem Sensor-Substrat (45) vorgesehen ist.

2. Biometrische Datenerkennungsvorrichtung nach Anspruch 1, wobei
die Gehäuse-Einheit (30) ein oberes Gehäuse (34) und ein unteres Gehäuse (36) aufweist, und
mindestens das obere Gehäuse (34) und die Band-Einheit (10) eingegossen sind.

3. Biometrische Datenerkennungsvorrichtung nach Anspruch 2, wobei
die Band-Einheit (10) aus einem ersten Material hergestellt ist, und das obere Gehäuse (34) aus einem zweiten Material, das härter als das erste Material ist, hergestellt ist.

4. Biometrische Datenerkennungsvorrichtung nach einem der Ansprüche 1-3, wobei die Gehäuse-Einheit (30) ein oberes Gehäuse (34) und ein unteres Gehäuse (36) aufweist, und
ein Öffnungs-Bereich (18), der das obere Gehäuse (34) aufnimmt, in der Band-Einheit (10) vorgesehen ist.

5. Biometrische Datenerkennungsvorrichtung nach einem der Ansprüche 1-4, wobei
ein biegsames Substrat (70), auf dem ein Licht-emittierender Bereich (72) angeordnet ist, auf einer hinteren Oberflächen-Seite der äußeren Oberfläche (31) der Gehäuse-Einheit (30) vorgesehen ist.

6. Biometrische Datenerkennungsvorrichtung nach einem der Ansprüche 1-5, wobei
die Gehäuse-Einheit (30) ein oberes Gehäuse (34) und ein unteres Gehäuse (36) aufweist,
das obere Gehäuse (34) einen Licht-emittierenden Fenster-Bereich für den Licht-emittierenden Bereich aufweist, und
das obere Gehäuse und der Licht-emittierende Fenster-Bereich (32) eingegossen sind.

7. Biometrische Datenerkennungsvorrichtung nach einem der Ansprüche 1-6, wobei
ein biegsames Substrat (70), auf dem eine Kommunikationsantenne angeordnet ist, auf einer hinteren Oberfläche der äußeren Oberfläche der Gehäuse-Einheit (30) vorgesehen ist.

8. Biometrische Datenerkennungsvorrichtung nach einem der Ansprüche 1-7, wobei
die Gehäuse-Einheit (30) ein oberes Gehäuse (34) und ein unteres Gehäuse (36) aufweist, und
eine Batterie (80) zwischen der Schaltplatine (160) und dem oberen Gehäuse (34) angeordnet ist.

9. Biometrische Datenerkennungsvorrichtung nach einem der Ansprüche 1-8, wobei
ein Vibrationserzeuger in der Gehäuse-Einheit (30) vorgesehen ist, und
der Vibrationserzeuger (180), bei einer ersten Seite und einer zweiten Seite der Schaltplatine (160), die einander gegenüberliegen, auf der ersten Seite vorgesehen ist.

10. Biometrische Datenerkennungsvorrichtung nach Anspruch 9, wobei
eine Körperbewegungs-Sensor-Einheit (170) auf der Schaltplatine (160) befestigt ist, und
eine Strecke zwischen dem Vibrationserzeuger (180) und der Körperbewegungs-Sensor-Einheit (170) länger als eine Strecke zwischen dem Vibrationserzeuger (180) und der Verarbeitungs-Einheit (200) ist.

11. Biometrische Datenerkennungsvorrichtung nach Anspruch 1, wobei
ein Vibrationserzeuger in der Gehäuse-Einheit vorgesehen ist,
die Gehäuse-Einheit ein oberes Gehäuse und ein unteres Gehäuse aufweist, und der Stoßdämpfer zwischen dem Vibrationserzeuger und dem unteren Gehäuse vorgesehen ist.

12. Biometrische Datenerkennungsvorrichtung nach einem der Ansprüche 1-11, wobei
die Gehäuse-Einheit (30) ein oberes Gehäuse (34) und ein unteres Gehäuse (36) aufweist,
die Sensor-Einheit (40) einen Licht-Empfangs-Bereich (42), einen Licht-emittierenden Bereich (44) und ein Licht-Übertragungs-Element (50) aufweist, das aus dem Licht-emittierenden Bereich (44) emittierendes Licht und einfallendes Licht zum Licht-Empfangs-Bereich (42) überträgt, und
das untere Gehäuse (36) und das Licht-sendende Element (50) eingegossen sind.

## Revendications

1. Appareil de détection d'information biométrique comprenant :
une unité en bande (10) ;
une unité de boîtier (30) qui est attachée à l'unité en bande (10);
une unité de capteur (40) qui est fournie dans l'unité de boîtier (30) ;
et
une unité de traitement (200) qui est fournie dans l'unité de boîtier (30) et détecte une information biométrique sur la base d'un signal de détection de l'unité de capteur (40), dans lequel
l'unité en bande (10) inclut une surface intérieure (11) ayant une première forme de surface incurvée sur un côté de celle-ci qui fait face à un objet test lors du port de l'appareil de détection d'information biométrique,
l'unité de boîtier (30) inclut une surface extérieure (31) ayant une deuxième forme de surface incurvée qui fait face à la surface intérieure (11) de l'unité en bande (10), et
un tableau de circuit (160) sur lequel l'unité de traitement (200) est montée et un substrat de capteur (45) sur lequel l'unité de capteur (40) est montée sont fournis dans l'unité de boîtier (30), **caractérisé en ce que** :
un amortisseur (92) est fourni entre le tableau de circuit (160) et le substrat de capteur (45).

2. Appareil de détection d'information biométrique selon la revendication 1, dans lequel
l'unité de boîtier (30) inclut un boîtier supérieur (34) et un boîtier inférieur (36), et
au moins le boîtier supérieur (34) et l'unité en bande (10) sont moulés par insertion.

3. Appareil de détection d'information biométrique selon la revendication 2, dans lequel
l'unité en bande (10) est formée d'un premier matériau, et le boîtier supérieur (34) est formé d'un deuxième matériau plus dur que le premier matériau.

4. Appareil de détection d'information biométrique selon l'une quelconque des revendications 1 à 3, dans lequel
l'unité de boîtier (30) inclut un boitier supérieur (34) et un boîtier inférieur (36), et
une portion de trou (18) qui accueille le boîtier supérieur (34) est fournie dans l'unité en bande (10).

5. Appareil de détection d'information biométrique selon l'une quelconque des revendications 1 à 4, dans lequel
un substrat flexible (70) sur lequel une portion émettrice de lumière (72) est disposée est fourni sur un côté de surface arrière de la surface extérieure (31) de l'unité de boîtier (30).

6. Appareil de détection d'information biométrique selon l'une quelconque des revendications 1 à 5, dans lequel
l'unité de boîtier (30) inclut un boîtier supérieur (34) et un boîtier inférieur (36),
le boîtier supérieur (34) inclut une portion de fenêtre émettrice de lumière pour la portion émettrice de lumière, et
le boîtier supérieur et la portion de fenêtre émettrice de lumière (32) sont moulés par insertion.

7. Appareil de détection d'information biométrique selon l'une quelconque des revendications 1 à 6, dans lequel
un substrat flexible (70) sur lequel une antenne de communication est disposée est fourni sur une surface arrière de la surface extérieure de l'unité de boîtier (30).

8. Appareil de détection d'information biométrique selon l'une quelconque des revendications 1 à 7, dans lequel
l'unité de boîtier (30) inclut un boîtier supérieur (34) et un boîtier inférieur (36), et
une pile (80) est disposée entre le tableau de circuit (160) et le boîtier supérieur (34).

9. Appareil de détection d'information biométrique selon l'une quelconque des revendications 1 à 8, dans lequel
un générateur de vibrations est fournis dans l'unité de boîtier (30), et
le générateur de vibrations (180) est fourni, entre un premier côté et un deuxième côté du tableau de circuit (160) qui sont opposés l'un à l'autre, sur le premier côté.

10. Appareil de détection d'information biométrique selon la revendication 9, dans lequel
une unité de capteur de mouvement de corps (170) est montée sur le tableau de circuit (160), et
une distance entre le générateur de vibrations (180) et l'unité de capteur de mouvement de corps (170) est plus longue qu'une distance entre le générateur de vibrations (180) et l'unité de traitement (200).

11. Appareil de détection d'information biométrique selon la revendication 1, dans lequel
un générateur de vibrations est fourni dans l'unité de boîtier,
l'unité de boîtier inclut un boîtier supérieur et un boîtier inférieur, et
un amortisseur est fourni entre le générateur de vibrations et le boîtier supérieur.

12. Appareil de détection d'information biométrique selon l'une quelconque des revendications 1 à 11, dans lequel
l'unité de boîtier (30) inclut un boîtier supérieur (34) et un boîtier inférieur (36),
l'unité de capteur (40) inclut une portion réceptrice de lumière (42), une portion émettrice de lumière (44), et un membre de transmission de lumière (50) qui transmet une lumière émise à partir de la portion émettrice de lumière (44) et une lumière incidente à la portion réceptrice de lumière (42), et
le boîtier inférieur (36) et le membre de transmission de lumière (50) sont moulés par insertion.
